# EUROPEAN PATENT SPECIFICATION

(11) **EP 1 732 649 B1**
(45) Date of publication and mention of the grant of the patent: **20.01.2010**
(21) Application number: 04821747.5
(22) Date of filing: 26.11.2004
(51) Int. Cl.: A61P 25/28, C12N 15/11, G01N 33/50

(54) **TREATMENT OF NEURODEGENERATIVE DISEASES BY THE USE OF LAPTM4B**
BEHANDLUNG VON NEURODEGENERATIVEN ERKRANKUNGEN DURCH VERWENDUNG VON LAPTM4B
TRAITEMENT DE MALADIES NEURODEGENERATIVES AU MOYEN DE LA LAPTM4B

(30) Priority: 26.03.2004 EP 04007447
(43) Date of publication of application: 20.12.2006
(73) Proprietor: CELLZOME AG, 69117 Heidelberg (DE)
(72) Inventor: HOPF, Carsten, 68165 Mannheim (DE); DREWES, Gerard, 69121 Heidelberg (DE); RUFFNER, Heinz, 4107 Ettlingen (CH)
(74) Representative: Huhn, Michael
(86) International application number: PCT/EP2004/013457
(87) International publication number: WO 2005/092443

(56) References cited:
- SHAO GEN-ZE ET AL: "Molecular cloning and characterization of LAPTM4B, a novel gene upregulated in hepatocellular carcinoma." ONCOGENE, vol. 22, no. 32, 7 August 2003 (2003-08-07), pages 5060-5069, XP002323512 ISSN: 0950-9232 cited in the application

## Description

The present invention relates to for use inhibitors of LAPTM4B in the treatment of neurodegenerative diseases.

Alzheimer's disease is a chronic condition that affects millions of individuals worldwide.

The brains of sufferers of Alzheimer's disease show a characteristic pathology of prominent neuropathologic lesions, such as the initially intracellular neurofibrillary tangles (NFTs), and the extracellular amyloid-rich senile plaques. These lesions are associated with massive loss of populations of CNS neurons and their progression accompanies the clinical dementia associated with AD. The major component of amyloid plaques are the amyloid beta (A-beta, Abeta or Aβ) peptides of various lengths. A variant thereof, which is the Aβ1-42-peptide (Abeta-42), is the major causative agent for amyloid formation. Another variant is the Aβ1-40-peptide (Abeta-40). Amyloid beta is the proteolytic products of a precursor protein, beta amyloid precursor protein (beta-APP or APP). APP is a type-I trans-membrane protein which is sequentially cleaved by several different membrane- associated proteases. The first cleavage of APP occurs by one of two proteases, alpha-secretase or beta-secretase. Alpha-secretase is a metalloprotease whose activity is most likely to be provided by one or a combination of the proteins ADAM-10 and ADAM-17. Cleavage by alpha-secretase precludes formation of amyloid peptides and is thus referred to as non-amyloidogenic. In contrast, cleavage of APP by beta-secretase is a prerequisite for subsequent formation of amyloid peptides. This secretase, also called BACE1 (beta-site APP-cleaving enzyme), is a type-I transmembrane protein containing an aspartyl protease activity (described in detail below).

The beta-secretase (BACE) activity cleaves APP in the ectodomain, resulting in shedding of secreted, soluble APPb, and in a 99-residue C-terminal transmembrane fragment (APP-C99). Vassar et al. (Science 286, 735-741) cloned a transmembrane aspartic protease that had the characteristics of the postulated beta-secretase of APP, which they termed BACE1.

Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids (aa) containing a 21-aa signal peptide followed by a prosequence domain spanning aa 22 to 45. There are alternatively spliced forms, BACE-I-457 and BACE-I-476. The extracellular domain of the mature protein is followed by one predicted transmembrane domain and a short cytosolic C-terminal tail of 24 aa. BACE1 is predicted to be a type 1 transmembrane protein with the active site on the extracellular side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (J. Biol. Chem. 279, 10542-10550). To date, no BACE1 interacting proteins with regulatory or modulatory functions have been described.

The APP fragment generated by BACE1 cleavage, APP-C99, is a substrate for the gamma-secretase activity, which cleaves APP-C99 within the plane of the membrane into an A-beta peptide (such as the amyloidogenic Aβ1-42 peptide), and into a C-terminal fragment termed APP intracellular domain (AICD) (Annu Rev Cell Dev Biol 19, 25-51). The gamma-secretase activity resides within a multiprotein complex with at least four distinct subunits. The first subunit to be discovered was presenilin (Proc Natl Acad Sci USA 94, 8208-13). Other known protein components of the gamma-secretase complex are Pen-2, Nicastrin and Aph-1a.

Despite recent progress in delineating molecular events underlying the etiology of Alzheimer's disease, no disease-modifying therapies have been developed so far. To this end, the industry has struggled to identify suitable lead compounds for inhibition of BACE1. Moreover, it has been recognized that a growing number of alternative substrates of gamma-secretase exist, most notably the Notch protein. Consequently, inhibition of gamma-secretase is likely to cause mechanism-based side effects. Current top drugs (e.g. Aricept®/donepezil) attempt to achieve a temporary improvement of cognitive functions by inhibiting acetylcholinesterase, which results in increased levels of the neurotransmitter acetylcholine in the brain. These therapies are not suitable for later stages of the disease, they do not treat the underlying disease pathology, and they do not halt disease progression.

Thus, there is an unmet need for the identification of novel targets allowing novel molecular strategies for the treatment of Alzheimer's disease. In addition, there is a strong need for novel therapeutic compounds modifying the aforementioned molecular processes by targeting said novel targets.

In a first aspect, the invention provides the use of a "LAPTM4B inhibitor selected from the group consisting of SiRMA, antisense oligonucleotides and ribozymes" for use in a method for the treatment of neurogenerative diseases.

In the context of the present invention, it has been surprisingly found that the Lysosomal associated transmembrane protein 4 beta protein (in the following LAPTM4B) forms part of different intracellular protein complexes which are involved in the aberrant processing of APP in Alzheimer's disease by gamma-secretase. Especially, it has been found that LAPTM4B is part of the APP-complex, of the APP-C99-complex and of the BACE1-complex, all molecules known to interact with gamma-secretase. These complexes are named after their respective key protein compound.

The identification of LAPTM4B as a key molecule in these complexes enables the use of molecules interacting with LAPTM4B for the treatment of neurodegenerative diseases. This is especially shown in the examples where it is demonstrated that siRNA directed against LAPTM4B results in attenuation of generation and/or secretion of Abeta-42.

In the context of the present invention, a "LAPTM4B interacting molecule" is a molecule which binds at least temporarily to LAPTM4B and which preferably modulates and particularly inhibits LAPTM4B activity.

Lysosomal-associated transmembrane protein 4 beta (LAPTM4B), a protein upregulated in hepatocellular carcinoma (Shao et al., 2003) is - based on primary sequence analysis - a "4-transmembrane spanning transporter" family member. The protein contains proline-rich regions that could bind SH3-domains at both N- and C-termini suggesting a possible scaffolding role.

Although the function of LAPTM4B is unknown, it is hypothesized (because of its strong sequence similarity with LAPTM4A) to function in the transport of nucleosides and/or nucleoside derivatives between the cytosol and the lumen of an intracellular membrane-bound compartment. The LAPTM4A protein is localized in lysosomes (Cabrita et al., 1999). Complementation experiments in yeast with the related gene and/or protein LAPTM4A (in the literature also referred to as MTP, Mtrp, KIAA0108) provide functional evidence for this notion (Hogue et al., 1996): Expression of recombinant LAPTM4A in yeast cells alters the sensitivity of these yeast cells to a heterogeneous group of compounds (e.g., antimetabolites, antibiotics, anthracyclines, ionophores, and steroid hormones) by changing the subcellular compartmentalization of these drugs (Hogue et al., 1999).

By analogy, LAPTM4B might thus be involved in regulation of the ion and/or metabolite milieu of the endosomal/lysosomal compartment that has recently been proposed as a candidate cellular site for APP processing (Pasternak et al., 2003). Targeting intracellular sites of Abeta generation is a very attractive Abeta-lowering strategy, as recent evidence suggests that, differing from the cleavage mechanism of other gamma-secretase substrates such as Notch, proteolytic processing of APP is independent of cell surface regulation by extracellular ligands and may instead be controlled by intracellularly (Kvotchev and Sudhof, 2004).

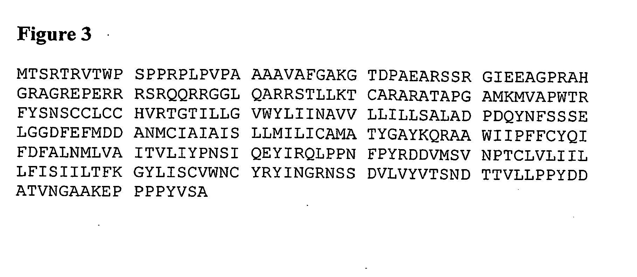

According to the present invention, the expression "LAPTM4B" does not only mean the protein as shown in Fig. 3, but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

The same applies also to all other proteins named in the present invention. Therefore, a name of given protein or nucleic acid does not only refer to the protein or nucleic acid as depicted in the sequence listing, but also to its functionally active derivative, or to a functionally active fragment thereof, or a homologue thereof, or a variant encoded by a nucleic acid that hybridizes to the nucleic acid encoding said protein under low stringency conditions, preferably under the conditions as mentioned above.

Since it is hypothesized that LAPTM4B and LAPTM4A have the same or at least similar functions (due to their strong sequence similarities, see Fig. 3), a "functionally active derivative" of LAPTM4B means in this case a derivate which exerts essentially the same activity as LAPTM4A (which is identical or similar to the function of LAPTM4B). LAPTM4A function (and therefore also the function of LAPTM4B and of its derivates, fragments and homologues) can be quantitatively determined by
a) their functional complementation of the nucleoside transport defect that is observed when exposing yeast cells expressing LAPTM4B or its "functionally active derivative" to compounds such as methotrexate and sulfanilamide
b) a drug sensitivity assay wherein the sensitivity of yeast cells expressing LAPTM4B or its "functionally active derivative" to compounds including but not limited to the ones mentioned by Hogue et al. (1999)
c) a cellular nucleoside transport assay wherein the uptake of radioactively labelled nucleosides (such as ¹⁴C nucleosides) is measured.

The above-mentioned functional assays for LAPTM4B- (or LAPTM4A-) activities are discussed in more detail in example 3.

In the case of other proteins, the term "functionally active" as used herein refers to a polypeptide, namely a fragment or derivative, having structural, regulatory, or biochemical functions of the protein according to the embodiment of which this polypeptide, namely fragment or derivative, is related to.

According to the present invention, the term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, stabilize, inhibit, suppress or destabilize the function of other molecules, stability, ability to localize to certain subcellular locations. Where applicable, said term also relates to the function of a protein complex in its broadest sense.

According to the present invention, the terms "derivatives" or "analogs of component proteins" or "variants" as used herein preferably include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions. It means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by suitable available in vitro assays as provided in the invention.

The term "fragment" as used herein refers to a polypeptide of at least 10, 20, 30, 40 or 50 amino acids of the component protein according to the embodiment. In specific embodiments, such fragments are not larger than 35, 100 or 200 amino acids.

The term "gene" as used herein refers to a nucleic acid comprising an open reading frame encoding a polypeptide of, if not stated otherwise, the present invention, including both exon and optionally intron sequences.

The terms " homologue" or "homologous gene products" as used herein mean a protein in another species, preferably mammals, which performs the same biological function as the a protein component of the complex further described herein. Such homologues are also termed "orthologous gene products". The algorithm for the detection of orthologue gene pairs from humans and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g., in Nature, 2001, 409:860-921. The homologues of the proteins according to the invention can either be isolated based on the sequence homology of the genes encoding the proteins provided herein to the genes of other species by cloning the respective gene applying conventional technology and expressing the protein from such gene, or by isolating proteins of the other species by isolating the analogous complex according to the methods provided herein or to other suitable methods commonly known in the art.

In a preferred embodiment of the present invention, the "LAPTM4B-interacting molecule" is a LAPTM4B-inhibitor.

According to the present invention the term "inhibitor" refers to a biochemical or chemical compound which preferably inhibits or reduces the activity of LAPTM4B. This can e.g. occur via suppression of the expression of the corresponding gene. The expression of the gene can be measured by RT-PCR or Western blot analysis. Furthermore, this can occur via inhibition of the activity, e.g. by binding to LAPTM4B.

Examples of such LAPTM4B-inhibitors are binding proteins or binding peptides directed against LAPTM4B, in particular against the active site of LAPTM4B, and nucleic acids directed against the LAPTM4B gene.

The term "nucleic acids against LAPTM4B" refers to double-stranded or single stranded DNA or RNA, or a modification or derivative thereof which, for example, inhibit the expression of the LAPTM4B gene or the activity of .LAPTM4B and includes, without limitation, antisense nucleic acids, aptamers, siRNAs (small interfering RNAs) and ribozymes..

Preferably, the inhibitor is selected from the group consisting of antibodies, antisense oligonucleotides, siRNA, low molecular weight molecules (LMWs), binding peptides, aptamers, ribozymes and peptidomimetics.

So-called "low molecular weight molecules" (in the following called "LMWs") are molecules which are not proteins, peptides, antibodies or nucleic acids, and which exhibit a molecular weight of less than 5000 Da, preferably less than 2000 Da, more preferably less than 1000 Da, most preferably less than 500 Da. Such LMWs may be identified in high-throughput procedures starting from libraries. Such methods are known in the art and are discussed in detail below.

These nucleic acids can be directly administered to a cell, or which can be produced intracellularly by transcription of exogenous, introduced sequences.

An "antisense" nucleic acid as used herein refers to a nucleic acid capable of hybridizing to a sequence-specific portion of a component protein RNA (preferably mRNA) by virtue of some sequence complementarity. The antisense nucleic acid may be complementary to a coding and/or noncoding region of a component protein mRNA. Such antisense nucleic acids that inhibit complex formation or activity have utility as therapeutics, and can be used in the treatment or prevention of disorders as described herein.

The antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides.

The nucleic acids, e.g. the antisense nucleic acids or siRNAs, can be synthesized chemically, e.g. in accordance with the phosphotriester method (see, for example, Uhlmann, E. & Peyman, A. (1990) Chemical Reviews, 90, 543-584). Aptamers are nucleic acids which bind with high affinity to a polypeptide, here LAPTM4B. Aptamers can be isolated by selection methods such as SELEX (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability.

Nucleic acids may be degraded by endonucleases or exonucleases, in particular by DNases and RNases which can be found in the cell. It is, therefore, advantageous to modify the nucleic acids in order to stabilize them against degradation, thereby ensuring that a high concentration of the nucleic acid is maintained in the cell over a long period of time (Beigelman et al. (1995) Nucleic Acids Res. 23:3989-94; WO 95/11910; WO'98/37240; WO 97/29116). Typically, such a stabilization can be obtained by introducing one or more internucleotide phosphorus groups or by introducing one or more non-phosphorus internucleotides.

Suitable modified internucleotides are compiled in Uhlmann and Peyman (1990), supra (see also Beigelman et al. (1995) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention. In general, the oligonucleotide can be modified at the base moiety, sugar moiety, or phosphate backbone.

The oligonucleotide may include other appending groups such as peptides, agents facilitating transport across the cell membrane (see, e.g., Letsinger et al., 1989, Proc. Natl. Acad. Sci. USA 86:6553-6556; Lemaitre et al., 1987, Proc. Natl. Acad. Sci. USA 84:648-652; International Patent Publication No. WO 88/09810) or blood-brain barrier (see, e.g., International Patent Publication No. WO 89/10134), hybridization-triggered cleavage agents (see, e.g., Krol et al., 1988, BioTechniques 6:958-976), or intercalating agents (see, e.g., Zon, 1988, Pharm. Res. 5:539-549).

In detail, the antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, D-mannosylqueosine, 5N-methoxycarboxymethyluracil, 5-methoxyuracil, 2-methyl-thio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

The use of suitable antisense nucleic acids is further described e.g. in Zheng and Kemeny (1995) Clin. Exp. Immunol., 100, 380-2; Nellen and Lichtenstein (1993) Trends Biochem. Sci., 18, 419-23, Stein (1992) Leukemia, 6, 697-74 or Yacyshyn, B. R. et al. (1998) Gastroenterology, 114, 1142).

In yet another embodiment, the oligonucleotide is a 2-a-anomeric oligonucleotide. An a-anomeric oligonucleotide (2-a-anomeric oder a-anomeric) forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, e.g., a peptide, hybridization-triggered cross-linking agent, transport agent, hybridization-triggered cleavage agent, etc.

Throughout the invention, oligonucleotides of the invention may be synthesized by standard methods known in the art, e.g., by use of an automated DNA synthesizer (such as are commercially avail-able from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. USA 85:7448-7451), etc.

In a specific embodiment, the antisense oligonucleotides comprise catalytic RNAs, or ribozymes (see, e.g., International Patent Publication No. WO 90/11364; Sarver et al., 1990, Science 247:1222-1225). In another embodiment, the oligonucleotide is a 2'-0-methylribonucleotide (Inoue et al., 1987, Nucl. Acids Res. 15:6131-6148), or a chimeric RNA-DNA analog (Inoue et al., 1987, FEBS Lett. 215:327-330).

In an alternative embodiment, the antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the component protein. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells.

Expression of the sequences encoding the antisense RNAs can be by any promoter known in the art to act in mammalian, preferably human, cells. Such promoters can be inducible or constitutive. Such promoters include, but are not limited to, the SV40 early promoter region (Bemoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. USA 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a component protein gene, preferably a human gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a component protein RNA it may contain and still form a stable duplex (or triplex, as the case may be). One skilled in the art can ascertain a tolerable degree of mismatch by use of standard procedures to determine the melting point of the hybridized complex.

The production and use of siRNAs as tools for RNA interference in the process to down regulate or to switch off gene expression, here LAPTM4B gene expression, is e.g. described in Elbashir, S. M. et al. (2001) Genes Dev., 15, 188 or Elbashir, S. M. et al. (2001) Nature, 411, 494. Preferably, siRNAs exhibit a length of less than 30 nucleotides, wherein the identity stretch of the sense strang of the siRNA is preferably at least 19 nucleotides.

Ribozymes are also suitable tools to inhibit the translation of nucleic acids, here the LAPTM4B gene, because they are able to specifically bind and cut the mRNAs. They are e.g. described in Amarzguioui et al. (1998) Cell. Mol. Life Sci., 54, 1175-202; Vaish et al. (1998) Nucleic Acids Res., 26, 5237-42; Persidis (1997) Nat. Biotechnol., 15, 921-2 or Couture and Stinchcomb (1996) Trends Genet., 12, 510-5.

Pharmaceutical compositions, comprising an effective amount of a nucleic acid in a pharmaceutically acceptable carrier, can be administered to a patient having a disease or disorder that is of a type that expresses or overexpresses a protein complex of the present invention.

The amount of the nucleic acid that will be effective in the treatment of a particular disorder or condition will depend on the nature of the disorder or condition, and can be determined by standard clinical techniques. Where possible, it is desirable to determine the nucleic acid cytotoxicity in vitro, and then in useful animal model systems, prior to testing and use in humans. It is described that pharmaceutical compositions comprising nucleic acids are administered via liposomes, microparticles, or microcapsules. It may be useful to use such compositions to achieve sustained release of the nucleic acids. In a specific aspect, it may be desirable to utilize liposomes targeted via antibodies to specific identifiable central nervous system cell types (Leonetti et al., 1990, Proc. Natl. Acad. Sci. U.S.A. 87:2448-2451; Renneisen et al., 1990, J. Biol. Chem. 265:16337-16342).

The term "binding protein" or "binding peptide" refers to a class of proteins or peptides which bind and inhibit LAPTM4B, and includes, without limitation, polyclonal or monoclonal antibodies, antibody fragments and protein scaffolds directed against LAPTM4B.

The term antibody or antibody fragment is also understood as meaning antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884), preferably produced with the help of a FAB expression library.

As an alternative to the classical antibodies it is also possible, for example, to use protein scaffolds against LAPTM4B, e.g. anticalins which are based on lipocalin (Beste et al. (1999) Proc. Natl. Acad. Sci. USA, 96, 1898-1903). The natural ligand-binding sites of the lipocalins, for example the retinol-binding protein or the bilin-binding protein, can be altered, for example by means of a "combinatorial protein design" approach, in such a way that they bind to selected haptens, here to LAPTM4B (Skerra, 2000, Biochim. Biophys. Acta, 1482, 337-50). Other known protein scaffolds are known as being alternatives to antibodies for molecular recognition (Skerra (2000) J. Mol. Recognit., 13, 167-187).

The procedure for preparing an antibody or antibody fragment is effected in accordance with methods which are well known to the skilled person, e.g. by immunizing a mammal, for example a rabbit, with LAPTM4B, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromatography. Monoclonal antibodies can, for example, be prepared in accordance with the known method of Winter & Milstein (Winter, G. & Milstein, C. (1991) Nature, 349, 293-299).

In detail, polyclonal antibodies can be prepared as described above by immunizing a suitable subject with a polypeptide as an immunogen. Preferred polyclonal antibody compositions are ones that have been selected for antibodies directed against a polypeptide or polypeptides of the invention. Particularly preferred polyclonal antibody preparations are ones that contain only antibodies directed against a given polypeptide or polypeptides. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a given polypeptide or polypeptides.

The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. For example, a recombinantly expressed and purified (or partially purified) protein is produced as described herein, and covalently or non-covalently coupled to a solid support such as, for example, a chromatography column. The column can then be used to affinity purify antibodies specific for the proteins from a sample containing antibodies directed against a large number of different epitopes, thereby generating a substantially purified antibody composition, i.e., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein, 1975, Nature 256:495-497, the human B cell hybridoma technique (Kozbor et al., 1983, Immunol. Today 4:72), the EBV-hybridoma technique (Cole et al., 1985, Monoclonal Antibodies and Cancer Therapy, Alan R. Liss, Inc., pp. 77-96) or trioma techniques. The technology for producing hybridomas is well known (see generally Current-Protocols in Immunology 1994, Coligan et al. (eds.) John Wiley & Sons, Inc., New York, NY). Hybridoma cells producing a monoclonal antibody are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, Bio/Technology 9:1370-1372; Hay et al., 1992, Hum. Antibod. Hybridomas 3:81-85; Huse et al., 1989, Science 246:1275-1281; Griffiths et al., 1993, EMBO J. 12:725-734.

Additionally, recombinant antibodies, such as chimeric and humanized monoclonal antibodies, comprising both human and non-human portions, which can be made using standard recombinant DNA techniques, are described in the invention. A chimeric antibody is a molecule in which different portions are derived from different animal species, such as those having a variable region derived from a murine mAb and a human immunoglobulin constant region. (See, e.g., Cabilly et al., U.S. Patent No. 4,816,567; and Boss et al., U.S. Patent No. 4,816,397, .) Humanized antibodies are antibody molecules from non-human species having one or more complementarily determining regions (CDRs) from the non-human species and a framework region from a human immunoglobulin molecule. (See, e.g., Queen, U.S. Patent No. 5,585,089,.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559); Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar, 1995, Int. Rev. Immunol. 13:65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope. (Jespers et al., 1994, Bio/technology 12:899-903).

Antibody fragments that contain the idiotypes of the complex can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments.

In the production of antibodies, screening for the desired antibody can be accomplished by techniques known in the art, e.g., ELISA (enzyme-linked immunosorbent assay). To select antibodies specific to a particular domain of the complex, or a derivative thereof, one may assay generated hybridomas for a product that binds to the fragment of the complex, or a derivative thereof, that contains such a domain. For selection of an antibody that specifically binds a given complex, or a derivative, or homologue thereof, but which does not specifically bind to the individual proteins of the complex, or a derivative, or homologue thereof, one can select on the basis of positive binding to the complex and a lack of binding to the individual protein components.

The foregoing antibodies can be used in methods known in the art relating to the localization and/or quantification of the given protein or proteins, e.g., for imaging these proteins, measuring levels thereof in appropriate physiological samples (by immunoassay), in diagnostic methods, etc. This hold true also for a derivative, or homologue thereof of a complex.

In a preferred embodiment, the LAPTM4B-inhibitor is either a siRNA with the sequences:
AACATGTTGGTTGCAATCACT (A) or AAACTCCATTCAGGAATACAT (B)

As discussed above, LAPTM4B is part of protein complexes which are involved in the regulation of gamma secretase activity and/or beta-secretase. Therefore, in a preferred embodiment, the LAPTM4B interacting molecule or inhibitor acts on a LAPTM4B molecule which is part of a protein complex, preferably of the APP-complex, of the BACE1-complex or of the APP-C99-compex.

Said protein complexes have been identified as assemblies of proteins interacting with the gamma-secretase substrates APP and APP-C99 and with beta-secretase protein.

As explained above, it has been surprisingly found in the context of the present invention that LAPTM4B is part of the protein complexes regulating proteolytic processing of APP, in particular by beta-secretase and/or gamma secretase activity. Therefore, in a preferred embodiment, the inhibitor or interacting molecule modulates the activity of beta- secretase and/or gamma secretase.

Throughout the invention, the term "modulating the activity of gamma secretase and/or beta secretase" includes that the activity of the enzyme is modulated directly or indirectly. That means that the LAPTM4B modulator may either bind also directly to either of these enzymes or, more preferred, may exert an influence on LAPTM4B which in turn, e.g. by protein-protein interactions or by signal transduction or via small metabolites, modulates the activity of either of these enzymes.
Throughout the invention, it is preferred that the beta secretase modulator inhibits the activity of beta secretase either completely or partially. Throughout the invention, the most preferred functional consequence of a LAPTM4B modulator is a reduction in Abeta-42 generation.

In the context of the present invention, "modulating the activity of gamma secretase and/or beta secretase" means that the activity is reduced in that less or no product is formed, most preferably that less or no Abeta-42 is formed, (partial or complete inhibition) or that the respective enzyme produces a different product (in the case of gamma-secretase e.g. Abeta-38 or other Abeta peptide species of shorter amino acid sequence - instead of Abeta-42) or that the relative quantities of the products are different (in the case of gamma-secretase e.g. the ratio of Abeta-40 to Abeta-42 is changed preferably increased). Furthermore, it is included that the modulator modulates either gamma secretase or beta-secretase or the activity of both enzymes.

With respect to the modulator of gamma secretase activity, it is preferred that this modulator inhibits gamma secretase activity. However, it is also preferred that the activity of gamma secretase is shifted in a way that the total amount of Abeta peptide species is unchanged but that more Abeta-38 is produced instead of Abeta-42.

Gamma secretase activity can e.g. measured by determining APP processing, e.g. by determining levels of Abeta peptide species produced, most importantly levels of Abeta-42 (see Example-section, infra).

To measure BACE1 activity, changes of the ratio between alpha- and beta-C-terminal APP fragments can be analyzed by Western Blotting (Blasko et al., J Neural Transm 111, 523); additional examples for BACE1 activity assays include but are not limited to: use of a cyclized enzyme donor peptide containing a BACE1 cleavage site to reconstitute and measure beta-galactosidase reporter activity (Naqvi et al., J Biomol Screen. 9, 398); use of quenched fluorimetric peptide substrates and fluorescence measurements (Andrau et al., J.Biol Chem 278, 25859); use of cell-based assays utilizing recombinant chimeric proteins, in which an enzyme (such as alkaline phosphatase) is linked via a stretch of amino acids, that contain the BACE1 recognition sequence, to a Golgi-resident protein (Oh et al., Anal Biochem, 323, 7); fluorescence resonance energy transfer (FRET)-based assays (Kennedy et al., Anal Biochen 319, 49); a cellular growth selection system in yeast (Luthi et al., Biochim Biophys Acta 1620, 167).

Preferably, the neurodegenerative disease is Alzheimer's disease.

The LAPTM4B interacting molecule may be used to prepare a pharmaceutical composition.

Therefore, the invention describes pharmaceutical compositions, which may be administered to a subject in an effective amount. In a preferred aspect, the therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a therapeutic , e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

Furthermore, it may be desirable to administer the pharmaceutical compositions locally to the area in need of treatment. This may be achieved by, for example, and not by way of limitation, local infusion during surgery topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Administration can be by direct injection at the site (or former site) of a malignant tumor or neoplastic or pre-neoplastic tissue.

It is further described that the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

The therapeutic can be delivered via a controlled release system. A pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). Polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). A controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In case where the therapeutic is a nucleic acid, preferably encoding a protein therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid therapeutic can be introduced intracellularly and incorporated by homologous recombination within host cell DNA for expression.

In general, the pharmaceutical compositions comprise a therapeutically effective amount of a therapeutic, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

The composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

The invention also describes a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits can also contain expression vectors encoding the essential components of the complex machinery, which components after being expressed can be reconstituted in order to form a biologically active complex. Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

The invention further describes a method of treatment, wherein an effective amount of a LAPTM4B-interacting molecule or inhibitor or of a pharmaceutical composition is administered to a subject suffering from a neurodegenerative disease, preferably Alzheimer's disease.

With respect to this method, all embodiments apply given above for the use of the invention.

The invention further relates to a method for identifying a gamma secretase modulator and/or beta-secretase modulator, comprising the following steps:
a. identifying of a LAPTM4B-interacting molecule by determining whether a given test compound is a LAPTM4B-interacting molecule,
b. determining whether the LAPTM4B-interacting molecule of step a) is capable of modulating gamma secretase activity and or beta-secretase activity.

In a preferred embodiment of the invention, in step a) the test compound is brought into contact with LAPTM4B and the interaction of LAPTM4B with the test compound is determined. Preferably, it is measured whether the candidate molecule is bound to LAPTM4B.

In a preferred embodiment of the invention, the LAPTM4B interacting molecule identified in step a) is first subjected to a LAPTM4B activity test as decribed supra (also see example 3) in order to find out whether it modulates, preferably inhibits LAPTM4B activity and is then subjected to process step b) (test for a Abeta-lowering effect).

The method of the invention is preferably performed in the context of a high throughput assay. Such assays are known to the person skilled in the art.

Test or candidate molecules to be screened can be provided as mixtures of a limited number of specified compounds, or as compound libraries, peptide libraries and the like.

Agents/molecules to be screened may also include all forms of antisera, antisense nucleic acids, etc., that can modulate complex activity or formation. Exemplary candidate molecules and libraries for screening are set forth below.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a LAPTM4B immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, LAPTM4B-fragments and/or analogs, especially peptidomimetics, are screened for activity as competitive or non-competitive inhibitors of the formation of a complex of LAPTM4B with another proteins, e.g. the proteins given in Table 1 (amount of complex or composition of complex) or LAPTM4B activity in the cell, which thereby inhibit complex activity or formation in the cell.

In one embodiment, agents that modulate (i.e., antagonize or agonize) LAPTM4B-activity or LAPTM4B-protein complex formation can be screened for using a binding inhibition assay, wherein agents are screened for their ability to modulate formation of a complex under aqueous, or physiological, binding conditions in which complex formation occurs in the absence of the agent to be tested. Agents that interfere with the formation of complexes of the invention are identified as antagonists of complex formation. Agents that promote the formation of complexes are identified as agonists of complex formation. Agents that completely block the formation of complexes are identified as inhibitors of complex formation.

Methods for screening may involve labeling the component proteins of the complex with radioligands (e.g., ¹²⁵I or ³H), magnetic ligands (e.g., paramagnetic beads covalently attached to photobiotin acetate), fluorescent ligands (e.g., fluorescein or rhodamine), or enzyme ligands (e.g., luciferase or β-galactosidase). The reactants that bind in solution can then be isolated by one of many techniques known in the art, including but not restricted to, co-immunoprecipitation of the labeled complex moiety using antisera against the unlabeled binding partner (or labeled binding partner with a distinguishable marker from that used on the second labeled complex moiety), immunoaffinity chromatography, size exclusion chromatography, and gradient density centrifugation. In a preferred embodiment, the labeled binding partner is a small fragment or peptidomimetic that is not retained by a commercially available filter. Upon binding, the labeled species is then unable to pass through the filter, providing for a simple assay of complex formation.

Methods commonly known in the art are used to label at least one of the component members of the complex. Suitable labeling methods include, but are not limited to, radiolabeling by incorporation of radiolabeled amino acids, e.g., ³H-leucine or ³⁵S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where one of the members of the complex is immobilized, e.g., as described infra, the free species is labeled. Where neither of the interacting species is immobilized, each can be labeled with a distinguishable marker such that isolation of both moieties can be followed to provide for more accurate quantification, and to distinguish the formation of homomeric from heteromeric complexes. Methods that utilize accessory proteins that bind to one of the modified interactants to improve the sensitivity of detection, increase the stability of the complex, etc., are provided.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular complexes can be assayed using routine protein binding assays to determine optimal binding conditions for reproducible binding.

The physical parameters of complex formation can be analyzed by quantification of complex formation using assay methods specific for the label used, e.g., liquid scintillation counting for radioactivity detection, enzyme activity for enzyme-labeled moieties, etc. The reaction results are then analyzed utilizing Scatchard analysis, Hill analysis, and other methods commonly known in the arts (see, e.g., Proteins, Structures, and Molecular Principles, 2nd Edition (1993) Creighton, Ed., W.H. Freeman and Company, New York).

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

Assays of agents (including cell extracts or a library pool) for competition for binding of one member of a complex (or derivatives thereof) with another member of the complex labeled by any means (e.g., those means described above) are provided to screen for competitors or enhancers of complex formation.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow complex formation.

After binding is performed, unbound, labeled protein is removed in the supernatant, and the immobilized protein retaining any bound, labeled protein is washed extensively. The amount of bound label is then quantified using standard methods in the art to detect the label as described, supra.

In another specific embodiments screening for modulators of the protein complexes/protein as provided herein can be carried out by attaching those and/or the antibodies as provided herein to a solid carrier.

The preparation of such an array containing different types of proteins, including antibodies) is well-known in the art and is apparent to a person skilled in the art (see e.g. Ekins et al., 1989, J. Pharm. Biomed. Anal. 7:155-168; Mitchell et al. 2002, Nature Biotechnol. 20:225-229; Petricoin et al., 2002, Lancet 359:572-577; Templin et al., 2001, Trends Biotechnol. 20:160-166; Wilson and Nock, 2001, Curr. Opin. Chem. Biol. 6:81-85; Lee et al., 2002 Science 295:1702-1705; MacBeath and Schreiber, 2000, Science 289:1760; Blawas and Reichert, 1998, Biomaterials 19:595; Kane et al., 1999, Biomaterials 20:2363; Chen et al., 1997, Science 276:1425; Vaugham et al., 1996, Nature Biotechnol. 14:309-314; Mahler et al., 1997, Immunotechnology 3:31-43; Roberts et al., 1999, Curr. Opin. Chem. Biol. 3:268-273; Nord et al., 1997, Nature Biotechnol. 15:772-777; Nord et al., 2001, Eur. J. Biochem. 268:4269-4277; Brody and Gold, 2000, Rev. Mol. Biotechnol. 74:5-13; Karlstroem and Nygren, 2001, Anal. Biochem. 295:22-30; Nelson et al., 2000, Electrophoresis 21:1155-1163; Honore et al.., 2001, Expert Rev. Mol. Diagn. 3:265-274; Albala, 2001, Expert Rev. Mol. Diagn. 2:145-152, Figeys and Pinto, 2001, Electrophoresis 2:208-216 and references in the publications listed here).

Protein or protein complexes can be attached to an array by different means as will be apparent to a person skilled in the art. Complexes can for example be added to the array via a TAP-tag (as described in WO/0009716 and in Rigaut et al., 1999, Nature Biotechnol. 10:1030-1032) after the purification step or by another suitable purification scheme as will be apparent to a person skilled in the art.

Optionally, the proteins of the complex can be cross-linked to enhance the stability of the complex. Different methods to cross-link proteins are well known in the art. Reactive endgroups of cross-linking agents include but are not limited to -COOH, -SH, -NH2 or N-oxysuccinamate.

The spacer of the cross-linking agent should be chosen with respect to the size of the complex to be cross-linked. For small protein complexes, comprising only a few proteins, relatively short spacers are preferable in order to reduce the likelihood of cross-linking separate complexes in the reaction mixture. For larger protein complexes, additional use of larger spacers is preferable in order to facilitate cross-linking between proteins within the complex.

It is preferable to check the success-rate of cross-linking before linking the complex to the carrier.

As will be apparent to a person skilled in the art, the optimal rate of cross-linking need to be determined on a case by case basis. This can be achieved by methods well known in the art, some of which are exemplary described below.

A sufficient rate of cross-linking can be checked f.e. by analysing the cross-linked complex vs. a non-cross-linked complex on a denaturating protein gel.

If cross-linking has been performed successfully, the proteins of the complex are expected to be found in the same lane, whereas the proteins of the non-cross-linked complex are expected to be separated according to their individual characteristics. Optionally the presence of all proteins of the complex can be further checked by peptide-sequencing of proteins in the respective bands using methods well known in the art such as mass spectrometry and/or Edman degradation.

In addition, a rate of crosslinking which is too high should also be avoided. If cross-linking has been carried out too extensively, there will be an increasing amount of cross-linking of the individual protein complex, which potentially interferes with a screening for potential binding partners and/or modulators etc. using the arrays.

The presence of such structures can be determined by methods well known in the art and include e.g. gel-filtration experiments comparing the gel filtration profile solutions containing cross-linked complexes vs. uncross-linked complexes.

Optionally, functional assays as will be apparent to a person skilled in the art, some of which are exemplarily provided herein, can be performed to check the integrity of the complex.

Alternatively, the proteins or the protein can be expressed as a single fusion protein and coupled to the matrix as will be apparent to a person skilled in the art.

Optionally, the attachment of the complex or proteins or antibody as outlined above can be further monitored by various methods apparent to a person skilled in the art. Those include, but are not limited to surface plasmon resonance (see e.g. McDonnel, 2001, Curr. Opin. Chem. Biol. 5:572-577; Lee, 2001, Trends Biotechnol. 19:217-222; Weinberger et al., 2000, 1:395-416; Pearson et al., 2000, Ann. Clin. Biochem. 37:119-145; Vely et al., 2000, Methods Mol. Biol. 121:313-321; Slepak, 2000, J. Mol Recognit. 13:20-26.

Exemplary assays useful for measuring the production of Abeta-40 and Abeta-42 peptides by ELISA include but are not limited to those described in Vassar R et al., 1999, Science, 286:735-41.

Exemplary assays useful for measuring the production of C-terminal APP fragments in cell lines or transgenic animals by western blot include but are not limited to those described in Yan R et al., 1999, Nature, 402:533-7.

Exemplary assays useful for measuring the proteolytic activity of beta- or gamma secretases towards bacterially expressed APP fragments in vitro (e.g. by modifying the expression of one or several interacting proteins in cells by means of RNAi (siRNA) and/or plasmids encoding the interacting protein(s)) of the BACE1-complex include but are not limited to those described in Tian G et al., 2002, J Biol Chem, 277:31499-505.

Exemplary assays useful for measuring transactivation of a Gal4-driven reporter gene (e.g. by modifying the expression of one or several interacting proteins in cells by means of RNAi (siRNA) and/or plasmids encoding the interacting protein(s)) of the BACE1-complex include but are not limited to those described in Cao X et al., 2001, Science, 293:115-20.

Any molecule known in the art can be tested for its ability to be an interacting molecule or inhibitor according to the present invention. Candidate molecules can be directly provided to a cell expressing the LAPTM4B-complex machinery, or, in the case of candidate proteins, can be provided by providing their encoding nucleic acids under conditions in which the nucleic acids are recombinantly expressed to produce the candidate protein.

The method of the invention is well suited to screen chemical libraries for molecules which modulate, e.g., inhibit, antagonize, or agonize, the amount of, activity of, or protein component composition of the complex. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention. Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et al., 1994, J. Med. Chem. 37:1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of -carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the alpha-carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, - amino isobutyric acid, 4-aminobutyric acid, Abu, 2-amino butyric acid; . -Abu, .-Ahx, 6-amino hexanoic acid; Aib, 2-amino isobutyric acid; 3-amino propionic acid; ornithine; norleucine; norvaline, hydroxyproline, sarcosine, citrulline, cysteic acid, t-butylglycine, t-butylalanine, phenylglycine, cyclohexylalanine, β-alanine, designer amino acids such as β-methyl amino acids, C-methyl amino acids, N-methyl amino acids, fluoro-amino acids and amino acid analogs in general. Furthermore, the amino acid can be D (dextrorotary) or L (levorotary).

In a specific embodiment, fragments and/or analogs of complexes of the invention, or protein components thereof, especially peptidomimetics, are screened for activity as competitive or non-competitive inhibitors of complex activity or formation.

In another embodiment of the present invention, combinatorial chemistry can be used to identify modulators of a the complexes. Combinatorial chemistry is capable of creating libraries containing hundreds of thousands of compounds, many of which, may be structurally similar. While high throughput screening programs are capable of screening these vast libraries for affinity for known targets, new approaches have been developed that achieve libraries of smaller dimension but which provide maximum chemical diversity. (See e.g., Matter, 1997, J. Med. Chem. 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest (in the instant invention, the protein complexes of the present invention and protein components thereof.) The fingerprints are compared with fingerprints obtained from other compounds known to react with the protein of interest to predict whether the library compound might similarly react. For example, rather than testing every ligand in a large library for interaction with a complex or protein component, only those ligands having a fingerprint similar to other compounds known to have that activity could be tested. (See, e.g., Kauvar et al., 1995, Chem. Biol. 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, Kurtz, Stanker and Skerritt (eds), 1995, AOAC: Washington, D.C., 305-312).

Kay et al. (1993, Gene 128:59-65) disclosed a method of constructing peptide libraries that encode peptides of totally random sequence that are longer than those of any prior conventional libraries. The libraries disclosed in Kay et al. encode totally synthetic random peptides of greater than about 20 amino acids in length. Such libraries can be advantageously screened to identify complex modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

In a preferred embodiment, the interaction of the test compound with LAPTM4B results in an inhibition of LAPTM4B-activity.

According to a preferred embodiment, in step b) the ability of the gamma-secretase and/or the beta-secretase to cleave APP is measured. This can be measured as indicated above.

Further, the invention also describes a method for preparing a pharmaceutical composition for the treatment of neurodegenerative diseases, preferably Alzheimer's disease, comprising the following steps:
a) identifying a gamma-secretase modulator and/or beta-secretase modulator, preferably inhibitor, according to the method of the invention, and
b) formulating the gamma-secretase and/or beta-secretase modulator, preferably inhibitor, to a pharmaceutical composition.

With respect to the pharmaceutical composition, all embodiments as indicated above apply also here.

In a preferred embodiment, this method further comprises the step of mixing the identified molecule with a pharmaceutically acceptable carrier as explained

The invention also describes a pharmaceutical composition comprising a LAPTM4B-inhibitor as defined above.

Furthermore, the invention describes a pharmaceutical composition obtainable by the above method for the preparation of a pharmaceutical composition.

The invention is describes the pharmaceutical composition for the treatment of a neurodegenerative disease such as Alzheimer's disease and related neurodegenerative disorders.

The invention also describes a method for treating or preventing a neurodegenerative disease, preferably Alzheimer's disease, comprising administering to a subject in need of such treatment or prevention a therapeutically effective amount of a pharmaceutical composition of the invention.

With respect to that method, all embodiments as described above for the use of the invention also apply.

The invention also relates to the use of a LAPTM4B-inhibitor selected from the group consisting of SiRMA, antisense oligonucleotides and ribozymes for the modulation, preferably inhibition of beta-secretase and/or gamma-secretase activity in vitro. For example, it is encompassed within the present invention to modulate, preferably inhibit beta-secretase and/or gamma-secretase activity in cell cultures by the LAPTM4B-interacting molecule. All embodiments with respect to the LAPTM4B inhibitor as described above also apply to this use of the invention.

The following examples will describe the subject-matter of the invention in more detail.

### Example 1

The TAP-technology, which is more fully described in EP 1 105 508 B1 and in Rigaut, et al., 1999, Nature Biotechnol. 17:1030-1032 respectively, was used and further adapted as described below for protein purification. Proteins were identified using mass spectrometry as described further below.

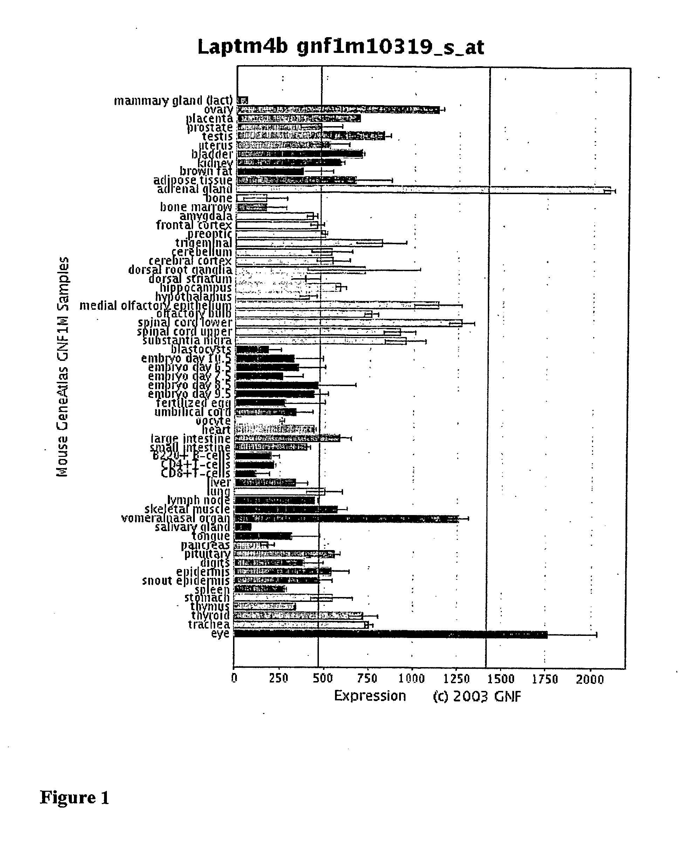

LAPTM4B was identified as a member of protein complexes with the TAP technology entry points APP695sw, APP-C99 and BACE1 (Figure 1)

### Part 1: Construction of TAP-tagged bait

The cDNAs encoding the complete ORF were obtained by RT-PCR. Total RNA was prepared from appropriate cell lines using the RNeasy Mini Kit (Qiagen). Both cDNA synthesis and PCR were performed with the SUPERSCRIPT One-Step RT-PCR for Long templates Kit (Life Technologies) using gene-specific primers. After 35-40 cycles of amplification PCR-products with the expected size were gel-purified with the MinElute PCR Purification Kit (Qiagen) and, if necessary, used for further amplification. Low- abundant RNAs were amplified by nested PCR before gel-purification. Restriction sites for NotI were attached to PCR primers to allow subcloning of amplified cDNAs into the retroviral vectors pIE94-N/C-TAP thereby generating N- or C-terminal fusions with the TAP-tag (Rigaut et al., 1999, Nature Biotechnol. 17:1030-1032). N-terminal tagging was chosen for the following baits/entry points: Presenilin 1, Presenilin 2, Aph-1a, Aph-1b Pen-2, APP, Tau, Fe65, Calsenilin. C-terminal tagging was chosen for the following baits/entry points: Nicastrin, Aph-1a, Aph-1b, BACE1 D215N, APP, APP695SW, APP-C99, Fe65, X11beta.
Clones were analyzed by restriction digest, DNA sequencing and by in vitro translation using the TNT T7 Quick Coupled Transcription/Translation System (Promega inc.). The presence of the proteins was proven by Western blotting using the protein A part of the TAP-tag for detection. Briefly, separation of proteins by standard SDS-PAGE was followed by semi-dry transfer onto a nitrocellulose membrane (PROTRAN, Schleicher&Schuell) using the MultiphorII blotting apparatus from Pharmacia Biotech. The transfer buffer consisted of 48 mM Tris, 39 mM glycine, 10% methanol and 0,0375% sodium dodecylsulfate. After blocking in phosphate-buffered saline (PBS) supplemented with 10% dry milk powder and 0,1% Tween 20 transferred proteins were probed with the Peroxidase-Anti-Peroxidase Soluble Complex (Sigma) diluted in blocking solution. After intensive washing immunoreactive proteins were visualized by enhanced chemiluminescence (ECL; Amersham Pharmacia Biotech).

### Part 2: Preparation of Virus and injection

As a vector, a MoMLV-based recombinant virus was used.

The preparation has been carried out as follows:

### 2.1. Preparation of Virus

293 gp cells were grown to 100% confluency. They were split 1:5 on poly-L-Lysine plates (1:5 diluted poly-L-Lysine [0.01% stock solution, Sigma P-4832] in PBS, left on plates for at least 10 min.). On Day 2, 63 microgram of retroviral Vector DNA together with 13 microgram of DNA of plasmid encoding an appropriate envelope protein were transfected into 293 gp cells (Somia, et al., 1999, Proc. Natl. Acad. Sci. USA 96:12667-12672; Somia, et al. 2000, J. Virol. 74:4420-4424). On Day 3, the medium was replaced with 15 ml DMEM +10% FBS per 15-cm dish. On Day 4, the medium containing viruses - (supernatant) was harvested (at 24 h following medium change after transfection). When a second collection was planned, DMEM 10 % FBS was added to the plates and the plates were incubated for another 24 h. All collections were done as follows: The supernatant was filtered through 0.45 micrometer filter (Corning GmbH, cellulose acetate, 431155). The filter was placed into konical polyallomer centrifuge tubes (Beckman, 358126) that are placed in buckets of a SW 28 rotor (Beckman). The filtered supernatant was ultracentrifuged at 19400 rpm in the SW 28 rotor, for 2 hours at 21 degree Celsius. The supernatant was discarded. The pellet containing viruses was resuspended in a small volume (for example 300 microliter) of Hank's Balanced Salt Solution [Gibco BRL, 14025-092], by pipetting up and down 100-times, using an aerosol-safe tip. The viruses were used for transfection as described below.

### 2.2. Infection

Cells that were infected were plated one day before into one well of a 6-well plate. 4 hours before infection, the old medium on the cells was replaced with fresh medium. Only a minimal volume was added, so that the cells are completely covered (e.g. 700 microliter).
During infection, the cells were actively dividing.

A description of the cells and their growth conditions is given further below ("3. Cell lines")

To the concentrated virus, polybrene (Hexadimethrine Bromide; Sigma, H 9268) was added to achieve a final concentration of 8 microgram/ml (this is equivalent to 2.4 microliter of the 1 milligram/ml polybrene stock per 300 microliter of concentrated retrovirus). The virus was incubated in polybrene at room temperature for 1 hour. For infection, the virus/polybrene mixture was added to the cells and incubated at 37 degree Celsius at the appropriate CO₂ concentration for several hours (e.g. over-day or overnight). Following infection, the medium on the infected cells was replaced with fresh medium. The cells were passaged as usual after they became confluent. The cells contain the retrovirus integrated into their chromosomes and stably express the gene of interest.

### 2.3. Cell lines

For expression, SKN-BE2 cells were used. SKN-BE2 cells (American Type Culture Collection-No. CRL-2271) were grown in 95% OptiMEM + 5% iron-supplemented calf serum.

### Part 3: Checking of expression pattern of TAP-tagged proteins

The expression pattern of the TAP-tagged protein was checked by immunoblot analysis and/or by immunofluorescence. Immunofluorescence analysis was either carried out according to No. 1 or to No. 2 depending on the type of the TAP-tagged protein.
Immunoblot analysis was carried out according to No. 3.

### 3.1 Protocol for the indirect Immunofluorescence staining of fixed mammalian cells for plasma membrane and ER bound proteins

Cells were grown in FCS media on polylysine coated 8 well chamber slides to 50% confluency. Then fixation of the cells was performed in 4% ParaFormAldehyde diluted in Phosphate Buffer Saline (PBS) solution (0.14M Phosphate, 0.1M NaCl pH 7.4). The cells were incubated for 30 minutes at room temperature in 300 microliters per well. Quenching was performed in 0.1M Glycine in PBS for 2x 20 minutes at room temperature. Blocking was performed with 1% Bovine Serum Albumin (BSA) in 0.3% Saponin + PBS for at least 1 hour at room temperature. Incubation of the primary antibodies was performed in the blocking solution overnight at +4°C. The proper dilution of the antibodies was determined in a case to case basis. Cells were washed in PBS containing 0.3% Saponin for 2x 20 minutes at room temperature. Incubation of the secondary antibodies is performed in the blocking solution. Alexa 594 coupled goat anti-rabbit is diluted 1:1000 (Molecular Probes). Alexa 488 coupled goat anti-mouse is diluted 1:1000 (Molecular Probes). DAPI was used to label DNA. If Phalloidin was used to label F-actin, the drug is diluted 1:500 and incubated with the secondary antibodies. Cells were then washed again 2x 20 minutes at room temperature in PBS. The excess of buffer was removed and cells were mounted in a media containing an anti-bleaching agent (Vectashield, Vector Laboratories).

### 3.2 Protocol for the indirect Immunofluorescence staining of fixed mammalian cells for non-plasma membrane bound proteins:

Cells were grown in FCS media on Polylysine coated 8 well chamber slides to 50% confluency. Fixation of the cells was performed in 4% ParaFormAldehyde diluted in Phosphate Buffer Saline (PBS) solution (0.14M Phosphate, 0.1M NaCl pH 7.4) for 30 minutes at Room Temperature (RT), 300 microliters per well. Quenching was performed in 0.1M Glycine in PBS for 2x 20 minutes at roon temperature. Permeabilization of cells was done with 0.5% Triton X-100 in PBS for 10 minutes at room temperature. Blocking was then done in 1% Bovine Serum Albumin (BSA) in 0.3% Saponin + PBS for at least 1 hour at RT (Blocking solution). Incubation of the primary antibodies was performed in the blocking solution, overnight at +4°C. The proper dilution of the antibodies has to be determined in a case to case basis. Cells were washed in PBS containing 0.3% Saponin, for 2x 20 minutes at RT. Incubation of the secondary antibodies was performed in the blocking solution. Alexa 594 coupled goat anti-rabbit is diluted 1:1000 (Molecular Probes), Alexa 488 coupled goat anti-mouse is diluted 1:1000 (Molecular Probes). DAPI was used to label DNA. If Phalloidin is used to label F-actin, the drug is diluted 1:500 and incubated with the secondary antibodies. Cells were washed 2x 20 minutes at RT in PBS. The excess of buffer was removed and cells were mounted in a media containing an anti-bleaching agent (Vectashield, Vector Laboratories).

### 3.3 Immunoblot analysis

To analyze expression levels of TAP-tagged proteins, a cell pellet (from a 6-well dish) was lyzed in 60 µl DNAse I buffer (5% Glycerol, 100 mM NaCl, 0.8 % NP-40 (IGEPAL), 5 mM magnesium sulfate, 100 µg/ml DNAse I (Roche Diagnostics), 50 mM Tris, pH 7.5, protease inhibitor cocktail) for 15 min on ice. Each sample was split into two aliquots. The first half was centrifuged at 13,000 rpm for 5 min. to yield the NP-40-extractable material in the supernatant; the second half (total material) was carefully triturated. 50 µg each of the NP-40-extractable material and the total material are mixed with DTT-containing sample buffer for 30 min at 50°C on a shaker and separated by SDS polyacrylamide gel electrophoresis on a precast 4-12% Bis-Tris gel (Invitrogen). Proteins were then transferred to nitrocellulose using a semi-dry procedure with a discontinuous buffer system. Briefly, gel and nitrocellulose membrane were stacked between filter papers soaked in either anode buffer (three layers buffer A1 (0.3 M Tris-HCl) and three layers buffer A2 (0.03 M Tris-HCl)) or cathode buffer (three layers of 0.03 M Tris-HCl, pH 9.4, 0.1 % SDS, 40 mM 0-aminocapronic acid). Electrotransfer of two gels at once was performed at 600 mA for 25 min. Transferred proteins were visualized with Ponceau S solution for one min to control transfer efficiency and then destained in water. The membrane was blocked in 5% non-fat milk powder in TBST (TBS containing 0.05% Tween-20) for 30 min at room temperature. It was subsequently incubated with HRP-coupled PAP antibody (1:5000 diluted in 5% milk/TBST) for 1 h at room temperature, washed three times for 10 min in TBST. The blot membrane was finally soaked in chemiluminescent substrate (ECL, Roche Diagnostics) for 2 min. and either exposed to X-ray film or analyzed on an imaging station.

### Part 4 Purification or protein complexes

Protein complex purification was adapted to the sub-cellular localization of the TAP-tagged protein and was performed as described below.

### 4.1 Lysate preparation for cytoplasmic proteins

About 1x10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of CZ lysis buffer (50 mM Tris-Cl, pH 7.4; 5 % Glycerol; 0,2 % IGEPAL; 1.5 mM MgCl₂; 100 mM NaCl; 25 mM NaF; 1 mM Na₃VO₄; 1 mM DTT; containing 1 tablet of EDTA-free Protease inhibitor cocktail (Complete™, Roche) per 25 ml of buffer) and homogenized by 10 strokes of a tight-fitted pestle in a dounce homogenizer. The lysate was incubated for 30 min on ice and spun for 10 min at 20,000g. The supernatant was subjected to an additional ultracentrifugation step for 1 h at 100,000g. The supernatant was recovered and rapidly frozen in liquid nitrogen or immediately processed further.

### 4.2 Lysate preparation for membrane proteins

About 1 x 10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of Membrane-Lysis buffer (50 mM Tris, pH 7.4; 7.5 % Glycerol; 1 mM EDTA; 150 mM NaCl; 25 mM NaF; 1 mM Na₃VO₄; 1 mM DTT; containing 1 tablet of EDTA-free Protease inhibitor cocktail (Complete™, Roche) per 25 ml of buffer) and homogenized by 10 strokes of a tight-fitted pestle in a dounce homogenizer. The lysate was spun for 10 min at 750g, the supernatant was recovered and subjected to an ultracentrifugation step for 1 h at 100,000g. The membrane pellet was resuspended in 7,5 ml of Membrane-Lysis buffer containing 0.8% n-Dodecyl-β-D-maltoside and incubated for 1 h at 4°C with constant agitation. The sample was subjected to another ultracentifugation step for 1h at 100,000g and the solubilized material was quickly frozen in liquid nitrogen or immediately processed further.

### 4.3 Lysate preparation for nuclear proteins

About 1x10⁹ adherent cells (average) were harvested with a cell scrapper and washed 3 times in ice-cold PBS (3 min, 550g). Collected cells were frozen in liquid nitrogen or immediately processed further. For cell lysis, the cell pellet was resuspended in 10 ml of Hypotonic-Lysis buffer (10 mM Tris, pH 7.4; 1.5 mM MgCl₂; 10 mM KCl; 25 mM NaF; 1 mM Na₃VO₄; 1 mM DTT; containing 1 tablet of EDTA-free Protease inhibitor cocktail (Complete™, Roche) per 25 ml of buffer) and homogenized by 10 strokes of a tight-fitted pestle in a dounce homogenizer. The lysate was spun for 10 min at 2,000g and the resulting supernatant (S1) saved on ice. The nuclear pellet (PI) was resuspended in 5 ml Nuclear-Lysis buffer (50 mM Tris, pH 7.4; 1.5 mM MgCl₂; 20 % Glycerol; 420 mM NaCl; 25 mM NaF; 1 mM Na₃VO₄; 1 mM DTT; containing 1 tablet of EDTA-free Protease inhibitor cocktail (Complete™, Roche) per 25 ml of buffer) and incubated for 30 min on ice. The sample was combined with S1, further diluted with 7 ml of Dilution buffer (110 mM Tris, pH 7.4; 0.7 % NP40; 1.5 mM MgCl₂; 25 mM NaF; 1 mM Na₃VO₄; 1 mM DTT), incubated on ice for 10 min and centrifuged at 100,000g for 1h. The final supernatant (S2) was frozen quickly in liquid nitrogen.

### 4.4 Tandem Affinity Purification

The frozen lysate was quickly thawed in a 37°C water bath, and spun for 20 min at 100,000g. The supernatant was recovered and incubated with 0.2 ml of settled rabbit IgG-Agarose beads (Sigma) for 2 h with constant agitation at 4°C. Immobilized protein complexes were washed with 10 ml of CZ lysis buffer (containing 1 Complete™ tablet (Roche) per 50 ml of buffer) and further washed with 5 ml of TEV cleavage buffer (10 mM Tris, pH 7.4; 100 mM NaCl; 0.1 % IGEPAL; 0.5 mM EDTA; 1 mM DTT). Protein- complexes were eluted by incubation with 5µl of TEV protease (GibcoBRL, Cat.No. 10127-017) for 1 h at 16°C in 150 µl TEV cleavage buffer. The eluate was recovered and combined with 0.2 ml settled Calmodulin affinity beads (Stratagene) in 0.2 ml CBP binding buffer (10 mM Tris, pH 7.4; 100 mM NaCl; 0,1 % IGEPAL; 2mM MgAc; 2mM Imidazole; 1mM DTT; 4 mM CaCl₂) followed by 1 h incubation at 4°C with constant agitation. Immobilized protein complexes were washed with 10 ml of CBP wash buffer (10 mM Tris, pH 7.4; 100 mM NaCl; 0,1 % IGEPAL; 1mM MgAc; 1mM Imidazole; 1mM DTT; 2 mM CaCl₂) and eluted by addition of 600 µl CBP elution buffer (10 mM Tris, pH 8.0; 5 mM EGTA) for 5 min at 37°C. The eluate was recovered in a siliconzed tube and lyophilized. The remaining Calmodulin resin was boiled for 5 min in 50 µl 4x Laemmli sample buffer. The sample buffer was isolated, combined with the lyophilised fraction and loaded on a NuPAGE gradient gel (Invitrogen, 4-12%, 1.5 mm, 10 well).

### Part 5 Protein Identification by Mass Spectrometry

### 5.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 min). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 5.2 Sample preparation prior to analysis by mass spectrometry

Gel plugs were extracted twice with 20 µl 1% TFA and pooled with acidified digest supernatants. Samples were dried in a a vaccum centrifuge and resuspended in 13 µl 1% TFA.

### 5.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass or QSTAR Pulsar, Sciex) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Tabelle 1: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 5.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999, Electrophoresis 20:3551-3567). Search criteria varied depending on which mass spectrometer was used for the analysis.

### Example 2: SiRNA-mediated knock-down of LAPTM4B

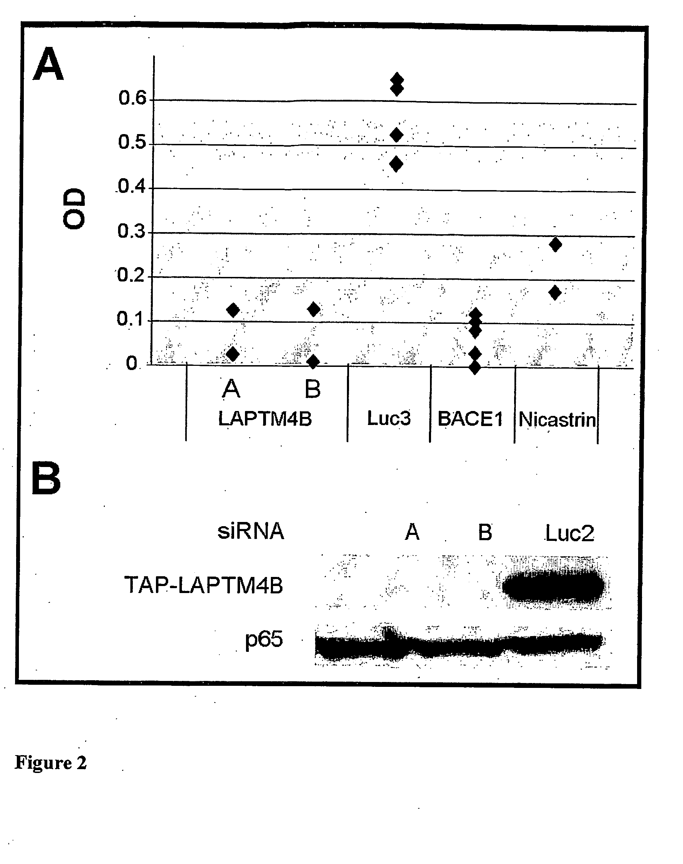

It was found that - like siRNAs directed against the known effectors of APP processing, BACE1 and nicastrin- the siRNAs targeting LAPTM4B cause significant attenuation of Aβ1-42 secretion, whereas the Luc3 siRNA has no effect (Figure 2A) - demonstrating that LAPTM4B plays a functional role in regulating the processing/secretion of APP.
It was further confirmed that the LAPTM4B-siRNAs did indeed interfere with the expression of LAPTM4B (Figure 2B).

### 2.1 siRNA knock-down and cellular Aβ1-42 assay

A RNAi gene expression perturbation strategy was employed for functional validation of LAPTM4B as an effector of APP processing: siRNAs A and B directed against LAPTM4B or siRNAs directed against known effectors of APP processing, BACE1 or nicastrin, or against unrelated Luc3 was transfected into SK-N-BE2 neuroblastoma cells expressing human APP695. SiRNAs for human LAPTM4B were synthesized by Dharmacon Research Inc..

The sequences of the siRNAs used for LAPTM4B are: AACATGTTGGTTGCAATCACT (A) and AAACTCCATTCAGGAATACAT (B).

Transfection of SK-N-BE2 cells was performed using LipofectAMINE 2000 (Invitrogen) following the manufacturer's instructions. Briefly, the cells were seeded at a density of 1.0 x 10⁴ cells in a final volume of 85 µl per 96-well 12-16 hrs prior to transfection. 25 nM of siRNAs were mixed with 8 µl Opti-MEM buffer (Gibco) and 60 ng carrier DNA, and the mixture was incubated for 20 minutes at room temperature before addition to the cells. 16 and 48 hrs post-transfection medium was replaced with 100 µl or 200 µl growth medium with or without serum, respectively. 72 hrs post-transfection 100 µl supernatants were harvested for Aβ1-42 ELISA (Innogenetics). The assay was performed following the manufacturer's instructions.

Knockdown efficiency of selected siRNAs was assessed at the protein level by co-transfecting siRNAs and corresponding TAP-tagged cDNA expression vectors (see below) or by using cell lines stably expressing the respective tagged protein of interest. 48 hrs post-transfection extracts were prepared, proteins separated by SDS-PAGE and transferred to nitrocellulose. Western blots were probed with antibodies directed against the TAP-tag or against unrelated p65.

### 2.2 Construction of TAP-tagged protein for validation of siRNAs

The cDNAs encoding the complete ORF were obtained by RT-PCR. Total RNA was prepared from appropriate cell lines using the RNeasy Mini Kit (Qiagen). Both cDNA synthesis and PCR were performed with the SUPERSCRIPT One-Step RT-PCR for Long templates Kit (Life Technologies) using gene-specific primers. After 35-40 cycles of amplification PCR-products with the expected size were gel-purified with the MinElute PCR Purification Kit (Qiagen) and, if necessary, used for further amplification. Low- abundant RNAs were amplified by nested PCR before gel-purification. Restriction sites for NotI were attached to PCR primers to allow subcloning of amplified cDNAs into the retroviral vectors pIE94-N/C-TAP thereby generating N-terminal fusions with the TAP-tag (Rigaut et al., 1999, Nature Biotechnol. 17:1030-1032).
Clones were analyzed by restriction digest, DNA sequencing and by in vitro translation using the TNT T7 Quick Coupled Transcription/Translation System (Promega inc.). The presence of the proteins was proven by Western blotting using the protein A part of the TAP-tag for detection. Briefly, separation of proteins by standard SDS-PAGE was followed by semi-dry transfer onto a nitrocellulose membrane (PROTRAN, Schleicher&Schuell) using the MultiphorII blotting apparatus from Pharmacia Biotech. The transfer buffer consisted of 48 mM Tris, 39 mM glycine, 10% methanol and 0,0375% sodium dodecylsulfate. After blocking in phosphate-buffered saline (PBS) supplemented with 10% dry milk powder and 0,1% Tween 20 transferred proteins were probed with the Peroxidase-Anti-Peroxidase Soluble Complex (Sigma) diluted in blocking solution. After intensive washing immunoreactive proteins were visualized by enhanced chemiluminescence (ECL; Amersham Pharmacia Biotech).

### Example 3: Determination of LAPTM4B activity

### 3.1 Functional complementation assay in yeast

In Saccharomyces cerevisiae suitable compounds such as methotrexate and sulfanilamide cause depletion of intracellular dTMP and growth arrest. Expression of LAPTM4 in yeast has been demonstrated to functionally complement this nucleoside transport defect (Hogue et al., 1996). Consequently, inhibitors of LAPTM4B can be identified by their ability to counter-act said functional complementation, i.e. by their ability to cause growth arrest in yeast strains engineered to express LAPTM4B and treated with suitable compounds such as methotrexate in the presence of extracellular nucleosides.

### 3.2 Drug sensitivity assay

A similar approach as described in a) can be used to measure sensitivity of yeast strains expressing LAPTM4B to a variety of drugs and drug-like molecules including but not limited to the ones mentioned by Hogue et al. (1999). Modulators of LAPTM4B, preferably inhibitors, can be identified as modulators of said drug sensitivity.

### 3.3 Cellular nucleoside transport assay

LAPTM4B lacking the C-terminus (as described for MTPβC in Hogue et al., 1996) can be expressed in plasma membranes of vertebrate cells including but not limited to *Xenopus laevis* oocytes. Uptake of radio-labeled nucleosides, such as 14C-labeled nucleosides, or other radio-labeled small molecules and/or metabolites, into such cells can be measured using methods available to a person skilled in the art.

### Example 4

### Modulation of Aβ1-42 generation/secretion by LAPTM4B modulators

SKNBE2 cells (or another suitable cell line) stably over-expressing human APP695 (SKNBE2/APP695) or a suitable mutant with enhanced beta-/gamma-secretase cleavage kinetics are plated in growth medium and serum-starved for 4 h the next morning. A LAPTM4B modulator, preferably inhibitor, diluted in serum-free medium, is then added and incubated for suitable periods of time. Cell supernatants are collected and levels of Aβ1-42 determined by ELISA (Innotest β-amyloid (1-42) from INNOGENETICS N.V., Belgium Innogenetics).

### The invention is described in more detail in the following figures:

- **Figure 1:**: Summary of the mouse tissue expression data as presented in GNF SymAtlas v0.8.0 (available in the public domain under "http://symatlas.gnf.org/terms.html", http://symatlas.gnf.org/SymAtlas/). It is shown that significant levels of LAPTM4B are expressed in the brain. The high expression of LAPTM4B in the brain supports the role of LAPTM4B in Alzheimer's disease.
- **Figure 2:**: SiRNA-mediated knock-down of LAPTM4B-expression attenuates secretion of Aβ1-42.

- **Fig. 2A:**: SiRNAs directed against BACE1, nicastrin, LAPTM4B (A or B) or Luc3 were transfected into SK-N-BE2 neuroblastoma cells over- expressing APP695. 48h after transfection growth medium was removed and cells were incubated over night in serum-free medium. Supernatants were collected and levels of Aβ1-42 determined by ELISA (Innogenetics). At least three independent experiments were performed in duplicate. A representative example is shown.
- **Fig. 2B:**: SiRNAs directed against LAPTM4B (A and B), but not a siRNA directed against unrelated Luc2, specifically reduce protein levels of co-transfected TAP-LAPTM4B. No effect was observed on expression levels of the unrelated protein p65.
- **Figure 3:**: Amino acid sequence of human LAPTM4B (LYSOSOMAL ASSOCIATED TRANSMEMBRANE PROTEIN 4 BETA), depicted in the one-letter-code

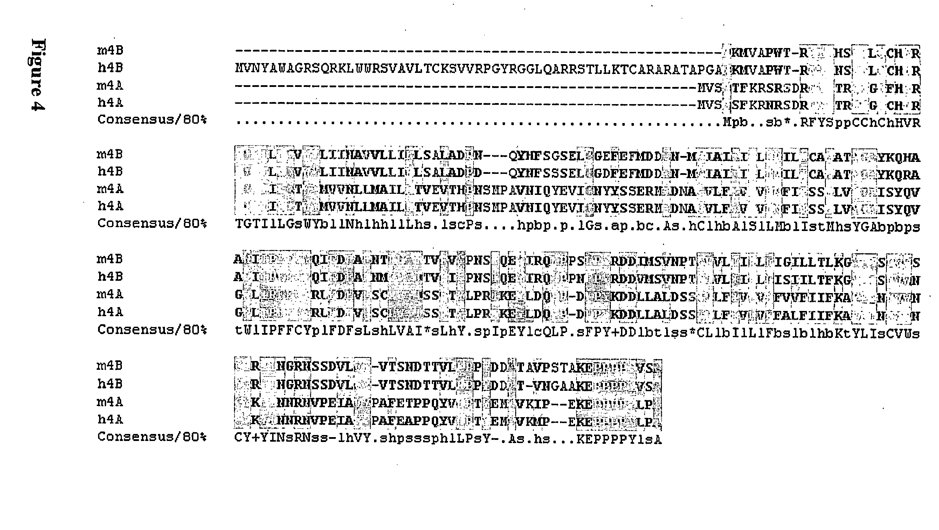

- **Figure 4:**: Multiple sequence alignment of mouse (m) and human (h) LAPTM4A and LAPTM4B

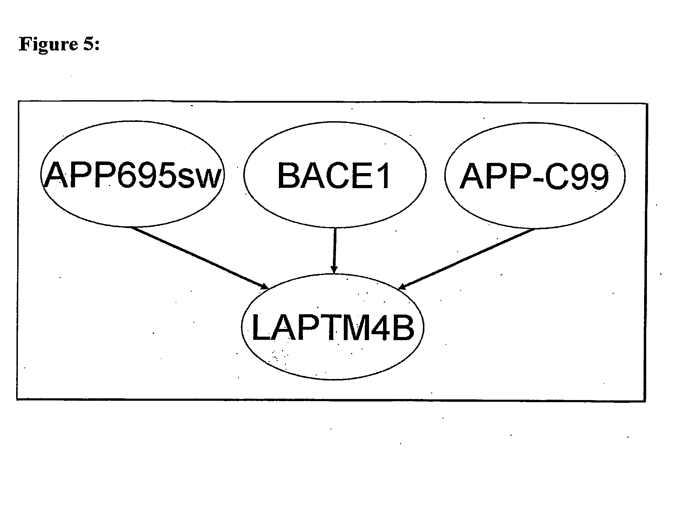

- **Figure 5:**: Schematic representation of TAP entry points (white) that LAPTM4B interacts with.

### References:

Cabrita MA, Hobman TC, Hogue DL, King KM, Cass CE (1999) Mouse transporter protein, a membrane protein that regulates cellular multidrug resistance, is localized to lysosomes. Cancer Res. 59(19):4890-7.
Hogue DL, Ellison MJ, Young JD, Cass CE (1996) Identification of a novel membrane transporter associated with intracellular membranes by phenotypic complementation in the yeast Saccharomyces cerevisiae. J Biol Chem. 271(16):9801-8.
Hogue DL, Kerby L, Ling V (1999) A mammalian lysosomal membrane protein confers multidrug resistance upon expression in Saccharomyces cerevisiae. J Biol Chem. 274(18):12877-82.
Khvotchev M, Sudhof TC (2004) Proteolytic Processing of Amyloid-{beta} Precursor Protein by Secretases Does Not Require Cell Surface Transport. J Biol Chem. 279(45):47101-47108.
Pasternak SH, Bagshaw RD, Guiral M, Zhang S, Ackerley CA, Pak BJ, Callahan JW, Mahuran DJ (2003) Presenilin-1, nicastrin, amyloid precursor protein, and gamma-secretase activity are co-localized in the lysosomal membrane. J Biol Chem. 278(29):26687-94.
Shao GZ, Zhou RL, Zhang QY, Zhang Y, Liu JJ, Rui JA, Wei X, Ye DX (2003) Molecular cloning and characterization of LAPTM4B, a novel gene upregulated in hepatocellular carcinoma. Oncogene 22(32):5060-9.

## Claims

1. A LAPTM4B-inhibitor selected from the group consisting of siRNA, antisense oligonucleotides and ribozymes for use in a method for the treatment of a neurodegenerative disease.

2. The inhibitor of claim 1, wherein LAPTM4B is part of an intracellular protein complex.

3. The inhibitor of any of claims 1 to 2, wherein the inhibitor modulates the activity of gamma-secretase and/or beta-secretase.

4. The inhibitor of any of claims 1 to 3, wherein the neurodegenerative disease is Alzheimer's disease.

5. A method for identifying a gamma-secretase and/or a beta-secretase modulator, comprising the following steps:
a. identifying of a LAPTM4B-interacting molecule by determining whether a given test compound is a LAPTM4B-interacting molecule,
b. determining whether the LAPTM4B-interacting molecule of step a) is capable of modulating gamma-secretase and/or beta-secretase activity.

6. The method of claim 5, wherein in step a) the test compound is brought into contact with LAPTM4B and the interaction of LAPTM4B with the test compound is determined.

7. The method of claim 6, wherein the interaction of the test compound with LAPTM4B results in an inhibition of LAPTM4B activity.

8. The method of any of claims 5 to 7, wherein in step b) the ability of the gamma-secretase and/or the beta-secrease to cleave APP is measured, preferably wherein the ability to produce Abeta 42 is measured.

9. Use of a LAPTM4B-inhibitor selected from the group consisting of siRNA, antisense oligonucleotides and ribozymes for the modulation of beta-secretase and/or gamma-secretase activity in vitro.

## Patentansprüche

1. LAPTM4B-Inhibitor, ausgewählt aus der Gruppe bestehend aus siRNA, Antisense-Oligonukleotiden und Ribozymen zur Verwendung in einem Verfahren für die Behandlung einer neurodegenerativen Erkrankung.

2. Inhibitor nach Anspruch 1, wobei LAPTM4B Teil eines intrazellulären Proteinkomplexes ist.

3. Inhibitor nach einem beliebigen der Ansprüche 1 bis 2, wobei der Inhibitor die Aktivität von gamma-Sekretase und/oder beta-Sekretase moduliert.

4. Inhibitor nach einem beliebigen der Ansprüche 1 bis 3, wobei die neurodegenerative Erkrankung Alzheimer-Krankheit ist.

5. Verfahren zum Identifizieren eines gamma-Sekretase- und/oder eines beta-Sekretase-Modulators, umfassend die folgenden Schritte:
a. Identifizieren eines LAPTM4B-interagierenden Moleküls durch Bestimmen, ob eine gegebene Testverbindung ein LAPTM4B-interagierendes Molekül ist,
b. Bestimmen, ob das LAPTM4B-interagierende Molekül aus Schritt a) zum Modulieren von gamma-Sekretase- und/oder beta-Sekretase-Aktivität fähig ist.

6. Verfahren nach Anspruch 5, wobei in Schritt a) die Testverbindung mit LAPTM4B in Kontakt gebracht wird, und die Interaktion von LAPTM4B mit der Testverbindung bestimmt wird.

7. Verfahren nach Anspruch 6, wobei die Interaktion der Testverbindung mit LAPTM4B in einer Inhibition von LAPTM4B-Aktivität resultiert.

8. Verfahren nach einem beliebigen der Ansprüche 5 bis 7, wobei in Schritt b) die Fähigkeit der gamma-Sekretase und/oder der beta-Sekretase, APP zu spalten, gemessen wird, wobei vorzugsweise die Fähigkeit gemessen wird, Abeta-42 zu erzeugen.

9. Verwendung eines LAPTM4B-Inhibitors, ausgewählt aus der Gruppe bestehend aus siRNA, Antisense-Oligonukleotiden und Ribozymen für die Modulierung von beta-Sekretase- und/oder gamma-Sekretase-Aktivität in vitro.

## Revendications

1. Un inhibiteur de LAPTM4B choisi dans le groupe comprenant siRNA, les oligonucléotides antisens et les ribozymes, destiné à être utilisé pour le traitement d'une maladie neurodégénérative.

2. L'inhibiteur de la revendication 1, dans lequel LAPTM4B fait partie d'un complexe protéinique intracellulaire.

3. L'inhibiteur selon une quelconque des revendications 1 à 2, dans lequel l'inhibiteur module l'activité de gamma-sécrétase et/ou de bêta-sécrétase.

4. L'inhibiteur selon une quelconque des revendications 1 à 3, dans lequel la maladie neurodégénérative est la maladie d'Alzheimer.

5. Un procédé d'identification d'un modulateur de béta-sécrétase et/ou de gamma-sécrétase, comprenant les étapes consistant à:
a. identifier une molécule interagissant avec LAPTM4B en déterminant si une molécule d'un composé testé donné interagit avec LAPTM4B,
b. déterminer si la molécule interagissant avec LAPTM4B de l'étape a) est capable de moduler l'activité de gamma-sécrétase et/ou de béta-sécrétase.

6. Un procédé selon la revendication 5, dans lequel dans l'étape a) le composé testé est mis au contact de LAPTM4B et on détermine l'interaction de LAPTM4B avec le composé testé.

7. Le procédé selon la revendication 6, dans lequel l'interaction du composé testé avec LAPTM4B conduit à une inhibition de l'activité de LAPTM4B.

8. Le procédé selon une quelconque des revendications 5 à 7, dans lequel on mesure dans l'étape b) la capacité de la gamma sécrétase et/ou de la béta-sécrétase à cliver APP, de préférence on mesure la capacité à produire l'Abéta 42.

9. Utilisation d'un inhibiteur de LAPTM4B choisi dans le groupe comprenant siRNA, les oligonucléotides antisens et les ribozymes pour la modulation de l'activité in vitro de béta-sécrétase et/ou de gamma-sécrétase.
